# EUROPEAN PATENT APPLICATION

(11) **EP 4 119 149 A1**
(43) Date of publication of application: **18.01.2023**
(21) Application number: 21386046.3
(22) Date of filing: 15.07.2021
(51) Int. Cl.: A61K 35/12, A61K 38/46, C12N 9/22, C12N 15/55, A61K 9/51

(54) **EXTRACELLULAR VESICLES COMPRISING A THERAPEUTIC CARGO**

(71) Applicant: Foundation For Research And Technology Hellas, 70013 Heraklion, Crete (GR)
(72) Inventor: Gkirtzimanaki, Katerina, 71305 Heraklion, Crete (GR); Niotis, Georgios, 71305 Heraklion, Crete (GR); Chatzidoukaki, Ourania, 70100 Heraklion, Crete (GR); Garinis, Georgios, 71414 Heraklion, Crete (GR); Goulielmaki, Evangelia, 71305 Heraklion, Crete (GR); Stratigi, Kalliopi, 71396 Heraklion, Crete (GR)
(74) Representative: Roukounas, Dimitrios

(57) **Abstract**

An extracellular vesicle loaded with a nuclease selected from S1 nuclease, Deoxyribonuclease I, Ribonuclease H, three prime repair exonuclease, or with messenger RNA encoding such nuclease and the use of the extracellular vesicle in the treatment of an inflammatory disease.

## Description

### TECHNICAL FIELD OF THE INVENTION

The present invention relates to extracellular vesicles (EVs) and their use in the prevention and/or treatment of inflammatory diseases.

### BACKGROUND OF THE INVENTION

Chronic inflammation plays a central role in some of the most challenging diseases of our time, including rheumatoid arthritis, cancer, heart disease, diabetes, asthma, and neurodegenerative disorders, including Alzheimer's and Parkinson's disease.

DNA damage triggers the activation of innate immune responses leading to chronic inflammation in mice and patients with inborn defects in DNA repair [Chatzinikolaou, G., Karakasilioti, I., and Garinis, G.A. (2014). DNA damage and innate immunity: links and trade-offs. Trends Immunol 35, 429-435.]. For example, the gradual accumulation of persistent DNA damage in mice carrying a DNA repair defect in adipocytes or macrophages was recently shown to trigger a chronic auto-inflammatory response leading to severe tissue degenerative changes and metabolic abnormalities associated with aging [Karakasilioti, I., Kamileri, I., Chatzinikolaou, G., Kosteas, T., Vergadi, E., Robinson, A.R., Tsamardinos, I., Rozgaja, T.A., Siakouli, S., Tsatsanis, C., et al. (2013). DNA damage triggers a chronic autoinflammatory response, leading to fat depletion in Nucleotide Excision Repair (NER) progeria. Cell Metab 18, 403-415.;Goulielmaki E, loannidou A, Tsekrekou M, Stratigi K, Poutakidou IK, Gkirtzimanaki K, Aivaliotis M, Evangelou K, Topalis P, Altmüller J, Gorgoulis VG, Chatzinikolaou G, Garinis GA. "Tissue-infiltrating macrophages mediate an exosome-based metabolic reprogramming upon DNA damage. Nat Commun. 2020 Jan 2;11(1):42], Furthermore, the nuclear factor kappa-light-chain-enhancer of activated B cells ((NF-κB) pathway, a prototypical pro-inflammatory signaling mechanism was found to be activated in animals that carry inborn a defect in NER and age in accelerated manner; notably, genetic depletion of the NF-κB p65 subunit or treatment with a pharmacological inhibitor of the NF-κB-activating kinase (IKK) delays the age-related symptoms seen in NER mutant animals [Tilstra, J.S., Robinson, A.R., Wang, J., Gregg, S.Q., Clauson, C.L., Reay, D.P., Nasto, L.A., St Croix, C.M., Usas, A., Vo, N., et al. (2012). NF-kappaB inhibition delays DNA damage-induced senescence and aging in mice. J Clin Invest 122, 2601-2612.].

Once established, DNA damage-driven chronic inflammation takes on a momentum of its own due to the amplification and feedback loops of the immune system leading to cellular malfunction, tissue degenerative changes and cancer.

Upon DNA damage, DNA fragments are known to accumulate in the cytoplasm of cells stimulating a type I interferon immune response via activation of the Cyclic GMP-AMP synthase (cGAS) -Stimulator of Interferon Genes (STING) pathway [Mackenzie, K.J., Carroll, P., Martin, C.A., Murina, O., Fluteau, A., Simpson, D.J., Olova, N., Sutcliffe, H., Rainger, J.K., Leitch, A., et al. (2017). cGAS surveillance of micronuclei links genome instability to innate immunity. Nature 548, 461-465.].

Extracellular vesicles (EVs) are vesicles secreted by cells into the extracellular space. The three main subtypes of EVs are microvesicles (MVs), exosomes, and apoptotic bodies, which are differentiated based upon their biogenesis, release pathways, size, content, and function.

Exosomes are small cell-derived vesicles of 30-200 nm in size that are found in all major biofluids and are important drivers of intercellular communication. Exosomes are released into the bloodstream and are known to discharge their content into far distant recipient cells and tissues to exert multiple physiological stimuli [Kojima, R., Bojar, D., Rizzi, G., Hamri, G.C., El-Baba, M.D., Saxena, P., Auslander, S., Tan, K.R., and Fussenegger, M. (2018). Designer exosomes produced by implanted cells intracerebrally deliver therapeutic cargo for Parkinson's disease treatment. Nat Commun 9, 1305. [Sterzenbach, U., Putz, U., Low, L.H., Silke, J., Tan, S.S., and Howitt, J. (2017). Engineered Exosomes as Vehicles for Biologically Active Proteins. Mol Ther 25, 1269-1278.].

The use of exosomes as advanced nanocarriers for the delivery of therapeutic biological molecules confers several innovative features and distinctive advantages over currently available anti-inflammatory drugs, liposomes or nanoparticles.

The EV-based delivery of a therapeutic cargo is non-immunogenic, avoids phagocytosis, avoids degradation by macrophages, avoids the endosomal pathway and lysosomal degradation, allows the EV therapeutic cargo to circulate for extended periods of time within the body, allows the EV therapeutic cargo to cross the blood brain barrier, has no biocompatibility issues, and minimal to no inherent toxicity issues, it is cost-effective for scaling up mass drug administration and offers the benefit of large scale deployment of personalized medicine.

### SUMMARY OF THE INVENTION

The present invention provides a novel therapeutic approach to combat inflammation triggered by cells exhibiting DNA damage.

Specifically, the present invention provides EVs loaded with a nuclease, or the messenger RNA (mRNA) encoding the nuclease, that can remove efficiently the cytoplasmic DNA moieties that accumulate in damaged cells.

The EVs of the present invention can be used in the treatment of a wide range of inflammatory diseases.

The present invention also provides a pharmaceutical composition comprising EVs that carry a cargo, which is a nuclease or the mRNA encoding the nuclease.

### BRIEF DESCRIPTION OF THE DRAWINGS

Figure 1 shows the fluorescence intensity (MFI) derived from the immunofluorescence detection of ssDNA in wild-type (wt) (1) and *Ercc1*^{*-*/*-*} (2) pancreata and primary pancreatic cells (PPCs).
Figure 2 shows the fluorescence intensity (MFI) derived from the immunofluorescence detection of R loops in wild-type (wt) or *Ercc1*^{*-*/*-*} cells upon various treatments.
Figure 3 shows the fluorescence intensity (MFI) derived from the immunofluorescence detection of ssDNA in the cytoplasm of wild-type (wt) or *Ercc1*^{*-*/*-*} cells upon various treatments
Figure 4 shows the fluorescence intensity (MFI) derived from the immunofluorescence detection of cytoplasmic ssDNAs or the *Ifnα* mRNA of *Ercc1*^{-/-} primary pancreatic cells (PPCs) exposed to naïve EVs, or to EVs loaded with S1 nuclease.
Figure 5 shows the mean fluorescence intensity (MFI) derived from the immunofluorescence detection of ssDNA in the cytoplasm of *Erec1*^{*-*/*-*} primary pancreatic cells from *Erec1*^{*-*/*-*} mice injected with naïve (control) EVs, or S1 nuclease-loaded EVs.
Figure 6 shows the fluorescence intensity (MFI) derived from the immunofluorescence detection of R loops and cytoplasmic ssDNAs in *Ercc1*^{*-*/*-*} PPCs exposed to naïve (control) (1), or extracellular vesicles loaded with recombinant RNase H (2).
Figure 7 shows *Ifnα* mRNA levels, α-SMA protein levels and STING protein levels from pancreata of *Ercc1^{-l-}* mice injected with naïve (1) or S1 nuclease-loaded EVs (2).
Figure 8 shows the bioactive Type I IFN levels in microglia cells treated with naive EVs and DNase I EVs as well as the percentage of Annexin V(+) Propidium Iodide (PI) (+) Purkinje cells in cerebellum slices derived from CX3CR1-*Ercc1*^{-/-} mice treated with naïve (control) or DNase I EVs.

### DETAILED DESCRIPTION OF THE INVENTION

The present invention provides an extracellular vesicle (EV) comprising a therapeutic cargo wherein the cargo comprises a nuclease or messenger RNA (mRNA) encoding the nuclease.

The term "extracellular vesicle" or "EV", as used herein, refers to a vesicle secreted by a cell into the extracellular space. Cells may be of several types, such as macrophages, murine fibroblasts, stem cells. An EV may be derived, for example, from a body fluid from a human or an animal from human or animal cell lines, cell cultures, or primary cultures. The surface of an EV comprises a lipid bilayer from the membrane of the donor cell, and the lumen of the EV is the same as the cytosol from the cell that produces the EV. Thus, the EV contains proteins, genetic material, lipids and carbohydrates of the producing cell.

EVs comprise microvesicles, exosomes, and apoptotic bodies, which are differentiated based upon their biogenesis, release pathways, size, content, and function. EVs can be isolated from a biological sample by various processes well known in the art, such as ultracentrifugation, density gradient centrifugation, size exclusion chromatography or polymer-based precipitation.

The term "exosome", as used herein, refers to an extracellular vesicle, having a size of 30-200 nm that is secreted from producing cells into the extracellular environment.

The term "cargo", as used herein, refers to a molecule, such as a nuclease or mRNA encoding the nuclease, which is loaded into an EV and which can be delivered to the cytoplasm of a cell.

The cargo of the EV of the present invention comprises a nuclease selected from S1 nuclease, Deoxyribonuclease (DNase) I, Ribonuclease (RNase) H, Three prime repair exonuclease (TREX) 1, or mRNA encoding such nuclease.

S1 nuclease catalyzes the degradation of primarily single-stranded DNA and RNA endonucleolytically to yield 5' phosphoryl-terminated products. The enzyme can occasionally introduce single-stranded breaks in double-stranded DNA or RNA, or DNA-RNA hybrids.

DNase I, is an endonuclease that digests single- and double-stranded DNA. It hydrolyzes phosphodiester bonds producing mono- and oligodeoxyribonucleotides with 5'-phosphate and 3'-OH groups.

RNase H is a family of non-sequence-specific endonuclease enzymes that catalyze the cleavage of RNA in an RNA/DNA substrate via a hydrolytic mechanism. RNases comprise subtypes H1 and H2.

TREX1 is a ubiquitous cellular 3'-5' exonuclease that degrades DNA fragments in the cytoplasm.

Preferably, the cargo of the EV comprises a nuclease selected from S1, DNase I, RNase H nuclease or the mRNA encoding such nuclease.

Preferably, the nuclease of the cargo, or the nuclease encoded by the mRNA is a recombinant nuclease.

The term "recombinant nuclease" refers to a nuclease encoded by recombinant DNA that has been cloned in an expression vector that supports expression of the gene and translation of messenger RNA.

Preferably, an EV according to the present invention has a size in the range of 20-1000nm. More preferably, the EV has a size in the range of 30 nm to 200 nm.

Preferably, the EV according to the present invention is an exosome.

According to a preferred embodiment, the EV comprises on its membrane a cell-penetrating peptide.

The term "cell-penetrating peptide", as used herein, refers to a short peptide, typically containing fewer than 30 amino acids, that facilitates cellular uptake of an EV and thus the release of its cargo into a cell. Cell-penetrating peptides as well as methods for their design and their production are well known in the art.

Preferably, the cell-penetrating peptide facilitates the uptake of the EV by specific cell types. For example, when the EV targets microglia cells, it may be coated with a cell penetrating peptide having the sequence CRHSQMTVTSRLRKLRSLWRR (SEQ ID NO: 1), which has been designed by combining a CD63 binding sequence [Gao X, Ran N, Dong X, Zuo B, Yang R, Zhou Q, Moulton HM, Seow Y, Yin H. Anchor peptide captures, targets, and loads exosomes of diverse origins for diagnostics and therapy. Sci Transl Med. 2018 Jun 6;10(444):eaat0195. doi:10.1126/scitranslmed.aat0195] and the *α*_{M}I-domain binding peptide CP05. Nataly P Podolnikova, Andriy V Podolnikov, Thomas A Haas, Valeryi K Lishko, Tatiana P Ugarova. Ligand recognition specificity of leukocyte integrin αMβ2 (Mac-1, CD11b/CD18) and its functional consequences. Biochemistry. 2015 Feb 17;54(6):1408-20.

Loading of an EV with the nuclease cargo can be achieved following methods well known in the art. Preferably, loading can be achieved through reversible permeabilization of the EV with saponin. For example, the EV can be treated with 0.2% saponin for 15 min at room temperature. Treatment with saponin enhances the loading capacity of the cargo into the EV compared with the simple incubation method, while it preserves the activity of the cargo and the structure of the EV.

According to an embodiment of the present invention, the EV is loaded with an mRNA cargo encoding a recombinant S1 or DNase I or RNase H or TREX1 nuclease. In such a case, the EV comprising the mRNA cargo may be produced through a modified cloning strategy for the generation of two vectors allowing to i. package mRNA encoding the nuclease (Vector 1 and 2) in the EV and ii. deliver the EV to targeted cells (Vector 1) [Kojima, R., Bojar, D., Rizzi, G., Hamri, G.C., El-Baba, M.D., Saxena, P., Auslander, S., Tan, K.R., and Fussenegger, M. (2018). Designer exosomes produced by implanted cells intracerebrally deliver therapeutic cargo for Parkinson's disease treatment. Nat Commun 9, 1305.].

Vector 1 contains a tetraspanin cluster of differentiation (CD)63 fused to L7Ae, and a Lysosome-associated membrane protein 2 (LAMP2)-Green Fluorescent Protein (GFP) fusion protein with a CD11b-specific peptide (CD11) N-terminally. The CD63-L7AE and CD11b-Lamp2-GFP chimeric proteins are expressed under Eukaryotic translation elongation factor 1 alpha (EF-1A) promoter. An Internal ribosome entry site (IRES) sequence ensures the simultaneous expression of both sequences. L7Ae is fused in the C-terminal of CD63 protein, an EV-specific tetraspanin. L7Ae ensures packaging of the desired mRNA as it binds the C/D box RNA structure [Rozhdestvensky, T.S., Tang, T.H., Tchirkova, I.V., Brosius, J., Bachellerie, J.P., and Huttenhofer, A. (2003). Binding of L7Ae protein to the K-turn of archaeal snoRNAs: a shared RNA binding motif for C/D and H/ACA box snoRNAs in Archaea. Nucleic Acids Res 31, 869-877.], which is conjugated with the nuclease encoding mRNA (Vector 2). CD63-L7Ae chimeric protein is then delivered in the inner-membrane of the EV retaining nuclease encoding mRNA. Lamp2 is an endocytic receptor fused with GFP in the C-terminus to track the engineered EV and serves as a carrier for the cell-specific targeting peptide, when present [Eskelinen, E.L., Illert, A.L., Tanaka, Y., Schwarzmann, G., Blanz, J., Von Figura, K., and Saftig, P. (2002). Role of LAMP-2 in lysosome biogenesis and autophagy. Mol Biol Cell 13, 3355-3368].

Vector 2 contains the nuclease, which is fused to a C/D box (3' Untraslated region) and is tagged with an Human influenza hemagglutinin (HA) and a FLAG tag N-terminally. The nuclease is cloned downstream of the Cytomegalovirus promoter without its native signal peptide to prevent secretion from targeted cells. NIH-3T3 cells are then co-transfected with Vector 1 and 2. The supernatant is collected 48-hrs post transfection and the EV fraction is isolated through differential centrifugation or through a previously established nickel-based isolation method [Gao, X., Ran, N., Dong, X., Zuo, B., Yang, R., Zhou, Q., Moulton, H.M., Seow, Y., and Yin, H. (2018). Anchor peptide captures, targets, and loads exosomes of diverse origins for diagnostics and therapy. Science translational medicine]

The EV of the present invention secretes its cargo in the cytoplasm of the cell it enters. When the recipient cell is inflamed the EV nuclease cargo, depending on its substrate, eliminates cytoplasmic single stranded DNA (ssDNA) or double stranded DNA (dsDNA). In the case of the RNase H nuclease, the EV nuclease cargo may also enter the nucleus to remove R-loops. Digestion of ssDNAs, dsDNAs or R-loops by the respective nuclease lowers substantially the pro-inflammatory response in the targeted cell eradicating inflammation. When the cargo is the nuclease mRNA (instead of the nuclease protein), then the respective mRNA is transferred and subsequently translated in the cytoplasm of recipient cells eliminating cytoplasmic ssDNAs and dsDNAs or nuclear R-loops, thereby lowering inflammation.

Thus, the present invention provides an EV as defined above for use in the treatment of an inflammatory disease in a subject, preferably a human subject. The inflammatory disease may be, for example, a chronic or an acute inflammatory disease and may affect one or more tissues, such as the brain, pancreas, kidney, liver, lung, skin or joint. Examples of inflammatory diseases which can be treated with the EV of the present invention include neuroinflammatory and neurodegenerative diseases, pancreatitis, kidney disease, liver inflammation, inflammatory arthritis and systemic lupus erythematosus.

The EV of the present invention is preferably administered to a subject in the form of a pharmaceutical composition. Thus, the present invention provides also a pharmaceutical composition comprising the EV of the present invention. The composition may be formulated, for example, for parenteral, intramuscular, intracerebral, intravenous, subcutaneous, transdermal, oral or inhalation administration. The composition may have different forms, such as solid or liquid forms, for example it can have the form of powder, solution, suspension or emulsion. The composition typically comprises the EV of the present invention and a pharmaceutically acceptable carrier. The pharmaceutically acceptable carrier may comprise one or more excipients, such as solvents, buffering agents, emulsifying agents, preservatives, antioxidants, diluents, binders and the like, which are well known in the art. Examples of solvents, include water, ethanol and isopropyl alcohol. Examples of buffering agents include citric acid monohydrate, acetic acid, sodium acetate and sodium hydrogen phosphate. Examples of emulsifying agents include sodium lauryl sulfate, lecithin, polysorbate and sorbitan monooleate. Examples of preservatives include sodium benzoate, benzyl alcohol and parahydroxy benzoic acids and their alkyl esters. Examples of antioxidants include sodium metabisulfite, butylated hydroxyanisole, butylated hydroxytoluene and ascorbic acid. Examples of diluents include lactose, sucrose, dextrose, mannitol and sorbitol. Examples of binders include hydroxypropylcellulose, hydroxyethylcellulose, dextrose, xylitol, polyvinylpyrrolidone and polyethylene glycol.

The therapeutically effective amount of the EVs according to the present invention and the frequency of their administration depend on various factors such as the disease to be treated, the route of administration or the form in which the EVs are administered. Typically, the therapeutically effective amount of the EV Is from 0.1 to 500 mg per kg of body weight of the subject. Preferably, the therapeutically effective amount is from 1 to 100 mg per kg of body weight. The EVs are typically administered once a day, although they can also be administered more or less frequently, for example, once weekly.

### EXAMPLES

### EXAMPLE 1

This example illustrates an EV-based strategy to deliver recombinant S1 nuclease to remove the accumulated ssDNAs in DNA repair-deficient Excision repair cross complementing group (*Ercc*)*1*^{*-*/*-*} PPCs.

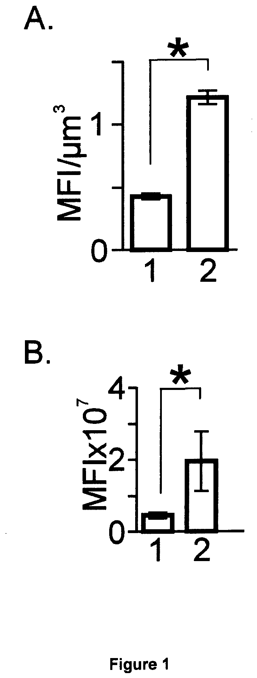

The pancreata and PPCs of (*Ercc*)*1*^{*-*/*-*} mice accumulate higher amounts of ssDNA than the wild type (wt) pancreata and PPCs as shown in Figure 1. Figure 1 A shows the mean fluorescence intensity (MFI) per mm³ of ssDNA in wild-type (1) and *Ercc1*^{*-*/*-*} (2) pancreata. Figure 1 B shows the MFI per mm³ of ssDNA in wild-type (1) and *Ercc1*^{*-*/*-*} (2) primary pancreatic cells (PPCs). Error bars indicate the standard error of the mean (S.E.M.) among n≥3 replicates. Horizontal connecting lines represent the statistical significance between wt and *Ercc1*^{*-*/*-*} samples. Significance (*) is set at P≤0.05 and was calculated by a two-tailed Student's t test.

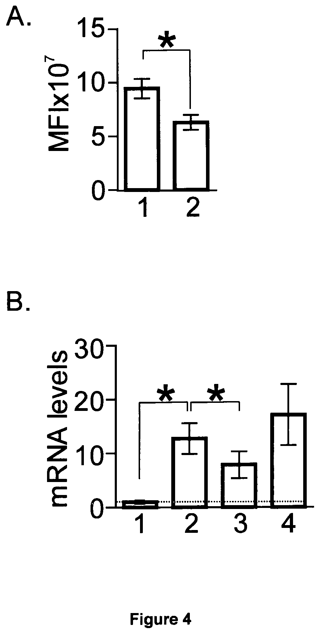

Thus, *Erec1*^{*-*/*-*} PPCs were exposed to naive EVs or S1 nuclease-loaded EVs. Importantly, it was found that the EV-mediated delivery of S1 nuclease substantially diminishes the accumulated ssDNAs in the cytoplasm of treated *Ercc1*^{*-*/*-*} PPCs leading to the decrease of the type I interferon *gene* ― alpha (*infα*) mRNA levels in these cells. The results are shown in Figure 4. Figure 4 A depicts the mean fluorescence intensity (MFI) per cell derived from the immunofluorescence detection of ssDNA in the cytoplasm of *Erec1*^{*-*/*-*} PPCs exposed to naïve (1), or extracellular vesicles loaded with recombinant S1 nuclease (2). Figure 4 B shows *Ifnα* mRNA levels in wt PPCs treated with EVs carrying the cytoplasmic nucleic acid fraction from wt. pancreata (1),or *Ercc1*^{*-*/*-*} pancreata (2), or *Ercc1*^{*-*/*-*} pancreata previously also treated with S1 nuclease (3) or *Ercc1*^{*-*/*-*} pancreata previously also treated with RNase A (4) as indicated. Error bars indicate the standard error of the mean (S.E.M.) among n≥3 replicates. Horizontal connecting lines represent the statistical significance between the indicated groups. Significance (*) is set at P≤0.05 and was calculated by a two-tailed Student's t test.

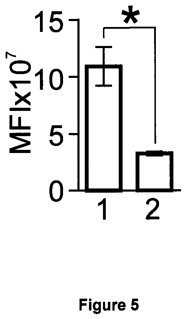

Having established the efficacy of the approach *in vitro,* P15 *Ercc1*^{*-*/*-*} mice were injected intraperitoneally for five consecutive days with naive or S1 nuclease-loaded EVs. It was found that treatment of P15 *Ercc1*^{*-*/*-*} mice with S1 nuclease-loaded EVs leads to the substantial removal of cytoplasmic ssDNAs, to the marked decrease in the mRNA levels of *ifna,* Matrix metallopeptidase *(mmp)9* and *p21* genes and to the substantial decrease in the protein levels of alpha-smooth muscle actin (a-SMA) and STING in *Ercc1*^{*-*/*-*} pancreata. The results are shown in Figure 5, Figure 6 and Figure 7.

Figure 5 depicts the mean fluorescence intensity (MFI) per cell derived from the immunofluorescence detection of ssDNA in the cytoplasm of *Ercc1*^{*-*/*-*} primary pancreatic cells from *Ercc1*^{*-*/*-*} mice injected with naive EVs (1) or S1 nuclease-loaded EVs (2). Error bars indicate the standard error of the mean (S.E.M.) among n≥3 replicates. The horizontal connecting line represents the statistical significance between the indicated groups. Significance (*) is set at P≤0.05 and was calculated by a two-tailed Student's t test.

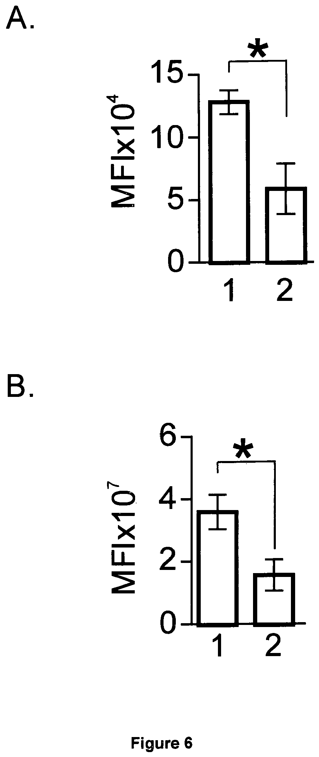

Figure 6 A depicts the mean fluorescence intensity (MFI) per cell nucleus derived from the immunofluorescence detection of R loops, by means of the S9.6 antibody staining, in *Ercc1*^{*-*/-} PPCs exposed to naïve (1), or extracellular vesicles loaded with recombinant RNase H (2). Figure 6 B depicts the mean fluorescence intensity (MFI) per cell nucleus derived from the immunofluorescence detection of ssDNAs in *Ercc1*^{*-*/*-*} PPCs exposed to naive (1), or extracellular vesicles loaded with recombinant RNase H (2). Error bars indicate the standard error of the mean (S.E.M.) among n≥3 replicates. The horizontal connecting line represents the statistical significance between the indicated groups. Significance (*) is set at P≤0.05 and was calculated by a two-tailed Student's t test.

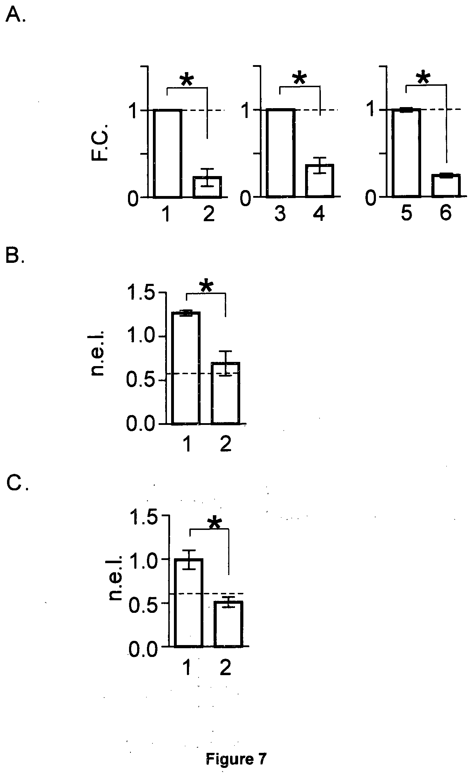

Figure 7 A shows *Ifnα* mRNA levels (y-axis) from pancreata of *Ercc1*^{*-*/*-*} mice injected with naive (1) or S1 nuclease-loaded extracellular vesicles (2) as compared to pancreata of untreated *Ercc1*^{*-*/*-*} mice (expressed as a fold change; fc). *Mmp9* mRNA levels from pancreata of *Ercc1*^{*-*/*-*} mice injected with naive (3) or S1 nuclease-loaded extracellular vesicles (4). *P21* mRNA levels from pancreata of wt. and *Ercc1*^{*-*/}*⁻mice* injected with naive (5) or S1 nuclease-loaded extracellular vesicles (6). The dashed line represents the wt. levels (n=5). Figure 7 B shows the β-TUB-normalized expression levels (n.e.l.) of α-SMA protein levels in whole-cell extracts from pancreata of *Ercc1*^{*-*/*-*} mice (n=5) injected with naive (1) or S1 nuclease-loaded EVs (2). The dashed line represents the wt. levels (n=5). Figure 7 C shows the β-TUB-normalized expression levels (n.e.l.) of STING protein levels in whole-cell extracts from pancreata of *Ercc1*^{*-*/*-*} mice (n=3) injected with naïve (1) or S1 nuciease-loaded EVs (2). The dashed line represents the wt. levels (n=5). Error bars indicate the standard error of the mean (S.E.M.) among n≥3 replicates. The horizontal connecting line represents the statistical significance between the indicated groups. Significance (*) is set at P≤0.05 and was calculated by a two-tailed Student's t test.

### Materials and methods:

**Animals.** The generation and characterization of ERCC1-deficient mice has been previously described (Niedernhofer, L.J., Garinis, G.A., Raams, A., Lalai, A.S., Robinson, A.R., Appeldoorn, E., Odijk, H., Oostendorp, R., Ahmad, A., van Leeuwen, W., et al. (2006). A new progeroid syndrome reveals that genotoxic stress suppresses the somatotroph axis. Nature 444, 1038-1043). Animals were kept on a regular diet and housed at the animal house of the Institute of Molecular Biology & Biotechnology of the Foundation for Research and Technology Hellas (IMBB-FORTH), which operates in compliance with the "Animal Welfare Act" of the Greek government, using the "Guide for the Care and Use of Laboratory Animals" as its standard. As required by Greek law, formal permission to generate and use genetically modified animals was obtained from the responsible local and national authorities. All animal studies were approved by an independent Animal Ethical Committee at FORTH.

**Primary cells and treatments.** Primary pancreatic cells (PPCs) were isolated from P15 animals. Briefly, pancreata were excised, minced and incubated in 2.5 mg/ml collagenase type IV at 37 °C for 15 min. After centrifugation, cells were resuspended and cultured in standard medium containing Dulbecco's Modified Eagle Medium (DMEM) supplemented with 10% Fetal Bovine Serum (FBS), 50 µg/ml streptomycin, 50 U/ml penicillin (Sigma) and 2 mM L glutamine (Gibco) for 2 days. For subsequent experiments, cells were rinsed with PBS, treated with Illudin S (30ng/ml, 3h, Santa Cruz) and cultured at 37°C prior to subsequent experiments. For the protein transfection experiments (Pierce^{™} Protein Transfection Reagent, Thermo), 10U of recombinant Mung bean S1 nuclease (100U/µl, Thermo) or RNase H (5U/µl; New England Biolabs) were used according to the manufacturer's instructions. Primary pancreatic cells were transfected with Lipofectamine 2000 (Invitrogen) and subsequently seeded in 6well or 24well plates, according to the manufacturer's protocol. As a non-targeting control, the AllStars negative (Qiagen) was used. For protein cell extracts, tissues were digested with collagenase (2.5mg/ml) at 37 °C for 15min. Collagenase was neutralized with 10% FBS and samples were further washed with 1x PBS/1%BSA.

**Purification and delivery of EVs.** For the delivery of EVs, 13-day-old *Er1*^{*-*/*-*} mice were injected intravenously every 24h for 5 days with EVs isolated from media of 10 × 10⁶ NIH-3T3 cells. EVs were purified using the differential ultracentrifugation protocol. Briefly, culture medium was centrifuged sequentially at 300g, (10min), 2000g (10min), and 10000g (30min) to remove dead cells and cell debris. EVs were purified with the final step of ultracentrifugation at 100000g for 2h. For functional experiments, the EVs used were purified from cells at a quantity of five times the number of recipient cells. S1 nuclease or RNase H or DNase 1 (1mg/ml, Santa Cruz) was loaded using 0.2% saponin. The EVs were then centrifuged for 1.5h at 100000g to ensure the incorporation of the nuclease, passed from a 0.2µm filter and were injected intraperitoneally to mice. For the isolation of cytoplasmic DNA, wt. and *Ercc1*^{*-*/*-*} pancreata were lysed with NP-40 lysis buffer (10mM Tris-HCl pH7.9, 10mM NaCl, 3mM MgCl₂, 0.5% NP-40, protease inhibitors). Cytoplasmic lysates were incubated with 0.2M NaCl and 0.1 mg/ml Proteinase K per 50ug protein for 2h at 65 °C. For the purification of DNA, the cytoplasmic extracts were loaded on phaselock tubes, With an equal volume of phenol/chloroform/isoamyl alcohol (25:24:1) and incubated for 5 min RT on a rotating wheel. The samples were centrifuged for 5min at RT. The aqueous phase was isolated and incubated for 3h at -80 ^{o}C with 0.2M NaCl, and 2,5 volumes cold 100% ethanol. The samples centrifuged for 25min at 4 ^{o}C. 80% ethanol added to the pellet and centrifuged for 5min at 4 ^{o}C. The DNA pellets were air-dried. 2.5ug of DNA were treated with 1ul S1 nuclease (100U/µl) or 1µl RNase A (100µg/µl) and incubated for 2h at 37 ^{o}C. The DNA was loaded to the EVs using 0.2% saponin and incubated with the PPCs for 24h.

**Immunofluorescence and antibodies.** For immunofluorescence experiments of mouse tissues, minced tissues or cells were fixed in 4% formaldehyde, permeabilized with 0.5% Triton-X and blocked with 1% BSA. After overnight incubation with primary antibodies, secondary fluorescent antibodies were added and DAPI was used for nuclear counterstaining. Samples were visualized with an SP8 TCS laser scanning confocal microscope (Leica) and scanned sections were analysed using the Fiji (ImageJ) software. Mouse livers, kidneys and pancreata were embedded in OCT compound, prior to frozen sectioning on a Cryotome CM 1850, permeabilization, blocking and immunostaining as described above. Antibodies against ssDNA (MAB3299 IF:1/70), dsDNA (MAB1293 IF:1/100), S9.6 (MABE1095) were from Millipore. Antibodies against α-SMA (ab5694, WB:1/500, IF:1/50) and b-tubulin (ab6046, WB:1/1000) were from Abcam. Antibody against STING (#13647S, WB:1/400) was from Cell Signaling Technology.

**Quantitative PCR.** Total RNA was isolated from wt. and *Ercc1*^{*-*/*-*} pancreata with the Total RNA isolation kit (Qiagen) as described by the manufacturer. Quantitative PCR (Q-PCR) was performed with a Biorad 1000-series thermal cycler according to the instructions of the manufacturer (Biorad); in case of the qPCR for the intronless *Ifna* gene, the RNA samples were treated with DNase (Promega). Primer sequences for the genes tested by qPCR are shown in the table below.

| **Gene name** | **Forward primer** | **Reverse primer** |
|---|---|---|
| *Mmp9* | GTCATTCGCGTGGATAAGGAG (SEQ ID NO: 2) | CACTGCAGGAGGTCGTAGG (SEQ ID NO. 3) |
| *P21* | TTGCACTCTGGTGTCTGAGC (SEQ ID NO: 4) | TCTGCGCTTGGAGTGATAGA (SEQ ID NO: 5) |
| *Ifnα* | CTGCTGGCTGTGAGGACATA (SEQ ID NO: 6) | GGCTCTCCAGACTTCTGCTC (SEQ ID NO: 7) |

### EXAMPLE 2

The gradual accumulation of irreparable DNA lesions triggers the formation of R-loops that causally contribute to the release and build-up of ssDNA fragments in the cytoplasm of DNA repair-deficient cells or cells treated with potent genotoxins e.g. illudin S, mitomycin C, Ultraviolet irradiation or cells that gradually accumulate DNA damage.

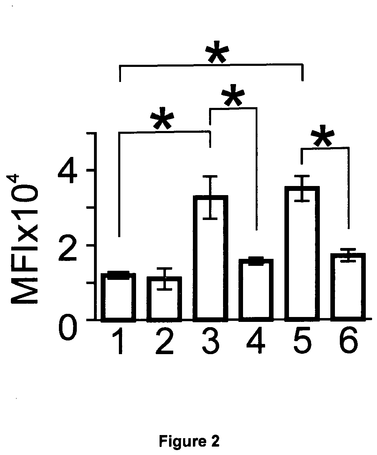

To exclude that the S9.6 immunofluorescence signal observed arises from dsRNA rather than RNA:DNA hybrids, PPCs were also transfected with the RNA:DNA hybrid-specific nuclease, RNase H; the latter removed RNA-DNA hybrids leading to the substantial decrease of R-loops in these cells. Figure 2 shows the mean fluorescence intensity (MFI) per cell nucleus derived from the immunofluorescence detection of R loops, by means of the S9.6 antibody staining, in untreated wild-type (wt) cells (1), in untreated wt. cells transfected with RNase H (2), in wt. cells treated with illudin S (3), in wt. cells treated with illudin S that were also transfected with RNase H (4), in *Ercc1*^{*-*/*-*} cells (5) and in *Ercc1*^{*-*/*-*} cells also transfected with RNase H (6). Error bars indicate the standard error of the mean (S.E.M.) among n≥3 replicates. Horizontal connecting lines represent the statistical significance between the indicated groups. Significance (*) is set at P≤0.05 and was calculated by a two-tailed Student's t test.

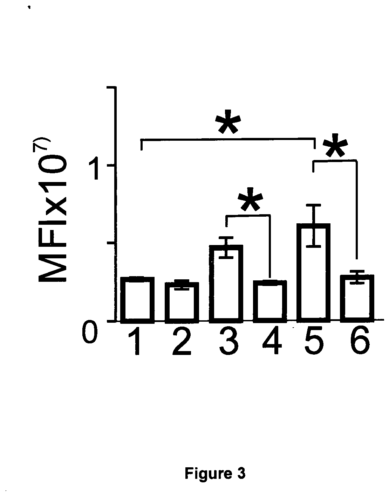

Next, it was sought to test whether DNA damage-driven R-loops causally contribute to the release of ssDNA moieties in the cytoplasm of Illudin S-treated and *Ercc1*^{*-*/*-*} PPCs. Consistently, it was found that ssDNAs are removed from the cytoplasm of Illudin S-treated or *Ercc1*^{*-*/*-*} PPCs when these cells are transfected with RNase H to eliminate R-loops. The results are shown in Figure 3 which depicts the mean fluorescence intensity (MFI) per cell derived from the immunofluorescence detection of ssDNA in the cytoplasm of untreated wt. cells transfected with recombinant *E*. *coli* RNase H (1) or not (2), in the cytoplasm of wt. cells treated with illudin S that were also transfected with recombinant *E. coli* RNase H (3) or not (4) and in *Ercc1*^{*-*/*-*} cells transfected with recombinant *E*. *coli* RNase H (5) or not (6). Error bars indicate the standard error of the mean (S.E.M.) among n≥3 replicates. Horizontal connecting lines represent the statistical significance between the indicated groups. Significance (*) is set at P≤0.05 and was calculated by a two-tailed Student's t test.

it was found that the R-loops derived ssDNAs in the cytoplasm stimulate a viral-like pro-inflammatory response in DNA repair-deficient *Ercc1*^{*-*/*-*} pancreata or in wild-type cells treated with genotoxins or in naturally aged 24-motnhs old pancreata.

The finding that DNA damage-driven R-loops causally contribute to the release of cytoplasmic ssDNAs in *Ercc1*^{*-*/*-*} pancreata prompted to test whether an EV-mediated delivery of recombinant RNase H could remove R-loops and the accumulated cytosolic ssDNAs in *Ercc1*^{*-*/*-*} pancreata. Intraperitoneal injections of P15 *Ercc1*^{*-*/*-*} mice with naive or RNase H-loaded EVs for two consecutive days revealed the substantial removal of R-loops leading to the marked elimination of cytoplasmic ssDNAs in *Ercc1*^{*-*/*-*} pancreata. The data are shown in Figure 6A and Figure 6B.

### Materials and methods:

**Purification and delivery of EVs.** EVs were isolated and loaded with recombinant RNase H as described in example 1. Treatment of cells or animals with EVs containing DNase I nuclease was performed as described in example 1.

### EXAMPLE 3

To dissect the impact of DNA damage in tissue-resident macrophages, animals homozygous for the floxed *Ercc1* allele (Ercc1^{F/F}) were intercrossed with mice carrying the CX3CR1-Cre transgene in an *Ercc1* heterozygous background (hereinafter denoted as Cx3CR1-*Ercc1*^{-/-} mice animals). CX3CR1 is a CX3C chemokine receptor 1 for fate-mapping studies of the tissue resident monocyte and macrophage compartment. Cx3CR1-*Ercc1*^{*-*/-}mice are born at the expected Mendelian frequency and present no developmental defects or other pathological features. Cx3CR1-*Ercc1*^{*-*/*-*} mice manifest signs of ataxia that progressively appeared around 4-months of age and became clearly evident in 6-months old animals. Cx3CR1-*Ercc1*^{-/-} mice animals stand with their hind limbs spread widely to maintain balance and walked with a wide gait and tremors. When Cx3CR1-*Ercc1*^{-/-} mice are suspended by their tails (a treatment that causes wild-type mice to extend and shake their hind limbs), animals kept their hind limbs often in a clasped position, indicating the loss of coordinated leg movement Rotarod assessment revealed that motor coordination declines progressively in Cx3CR1-*Ercc1*^{*-*/*-*} mice becoming clearly visible within 6 months after birth, when hind limb coordination deficiency is apparent in ~70% of the Cx3CR1-*Ercc1*^{*-*/*-*}animals compared to littermate controls. Beginning 8-months of age, Cx3CR1-*Ercc1*^{-/-} mice animals develop kyphosis and fine tremor to front legs. The premature onset of neurodegenerative features in Cx3CR1-*Ercc1*^{*-*/*-*} mice animals prompted us to assess the morphological and phenotypic characteristics of CNS-resident macrophages i.e. microglia cells.
In Cx3CR1-*Ercc1*^{-/-} brains, it was found that γ-H2A.X and pATM proteins accumulate in the cytoplasm of CD11b⁺ cells where they colocalize with DAPI+ chromatin fragments in Cx3CR1-*Ercc1*^{-/-} freshly isolated microglial cells.

The finding that DNA damage causally contributes to the release of cytoplasmic dsDNAs in Cx3CR1-*Ercc1*^{-/-} microglia cells prompted to test whether an EV-mediated delivery of recombinant DNase I could remove the accumulated cytosolic dsDNAs in Cx3CR1-*Ercc1*^{-/-}microglia cells, thereby lowering the proinflammatory response.

To do so, microglia cells were freshly isolated with magnetic bead-positive selection from mice carrying an inborn *Ercc1* defect only in tissue-resident macrophages (CX3CR1-*Ercc1*^{-/-}) and were treated with EVs loaded with DNase I for 4 hours. DAPI staining revealed the efficient removal of cytoplasmic chromatin fragments and that LaminB1 perinuclear localization is restored.

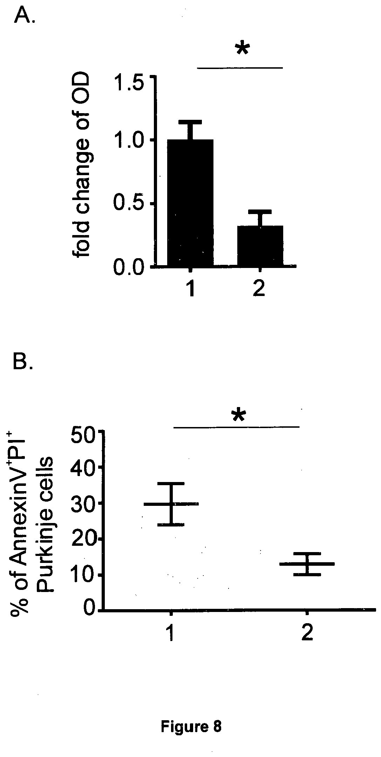

Having established that the exposure of Cx3CR1-*Ercc1*^{-/-} microglia cells to DNase I eliminates cytoplasmic dsDNAs *in vitro,* it was next embarked on a more physiological approach. Cx3CR1-*Ercc1*^{-/-} mice manifest signs of ataxia that progressively appear within 4-months of age. Intranasal delivery of EVs loaded with DNase I in 3-months old Cx3CR1-*Ercc1*^{*-*/*-*} mice twice a week for 6 weeks, led to the substantial removal of dsDNAs from Cx3CR1-*Ercc1*^{-/-} microglia cells and an improvement of motor coordination in the latency period (the time it takes a mouse to fall off the rod rotating under continuous acceleration from 5 to 70 rpm) measured during the Rotarod assay. In addition, it was found that treatment of Cx3CR1-*Ercc1*^{-/-} mice with EVs loaded with DNase I led to the significant decrease in Purkinje cell death (as assessed by FACS analysis of Annexin V staining of calbindin+ cells in the cerebellum) and microglia activation (as assessed by FACS analysis of MHCII+CD86+ cells in the cerebellum). Moreover, that treatment of Cx3CR1-*Ercc1*^{-/-}mice with EVs loaded with DNase I led to the decrease of bioactive Type I IFNs. The data are shown in Figure 8.

Figure 8 A depicts the bioactive Type I IFN levels in microglia cells treated with naive EVs (1) and DNase I EVs (2). Figure 8 B depicts the percentage of Annexin V(+) Propidium Iodide (PI) (+) Purkinje cells in cerebellum slices derived from CX3CR1-*Ercc1*^{-/-} mice treated with naïve control EVs (1) or with DNase I EVs (2).Error bars indicate the standard error of the mean (S.E.M.) among n≥3 replicates. The horizontal connecting line represents the statistical significance between the indicated groups. Significance (*) is set at P≤0.05 and was calculated by a two-tailed Student's t test.

### Materials and methods:

**Animals.** Animals homozygous for the floxed *Ercc1* allele (Ercc1F/F) were intercrossed with mice carrying the CX3CR1-Cre transgene in an *Ercc1* heterozygous background. Mice lacking the CX3CR1-Cre transgene in an *Ercc1* homozygous background were considered controls (wt). All animals were maintained in grouped cages in a temperature-controlled virus-free facility on a 12-h light/dark cycle and fed a normal diet (Lactamin, Stockholm, Sweden). Mice had access to water ad libitum. This work received ethical approval by and independent Animal Ethical Committee at the IMBB-FORTH. All relevant ethical guidelines for the work with animals were adhered to during this study.

**EV isolation, labelling and loading.** EVs were purified using the differential ultracentrifugation protocol. Briefly culture medium was centrifuged sequentially at 300 g, (10 min), 2000 g (10 min), and 10000 g (30 min) to remove dead cells and cell debris. Extracellular vesicles were isolated with an ultracentrifugation at 100000 g for 2 hrs and were then purified using a 90% to 10% sucrose gradient. Purified EVs were collected after a final ultracentrifugation at 100000 g for 2 hrs. All ultracentrifugations were performed at 4°C. For PKH67 staining, EVs were incubated with PKH67 (500 mL 0.2 mM) for 5 min at room temperature. Labelled EVs were diluted in 500 mL 1% BSA, and then pelleted at 100.000 g, washed with 1 mL PBS to remove excess dye, re-suspended in 1 mL PBS and then pelleted at 100.000 g before final re-suspension. For the extravesicular labelling of EVs against antibodies and fluorochromes, brain lavage ― derived EVs were incubated with both of them at dark, (20 min) RT. For intravesicular stainings, EVs were fixed with 0.01% formaldehyde 19 (15 min) at 4°C, washed with 0,2% saponin, 5% BSA in 1XPBS (permeabilization/blocking buffer) and finally isolated after ultracentrifugation at 4oC, 100000 g for 2 h. EVs were re-suspended and incubated in blocking buffer for 30 min, 4oC and then the immunofluorescent primary antibody was added. EVs were incubated with the primary antibody for 45 min, 4°C and finally the secondary fluorescent antibody was added and DAPI was used for nuclear counterstaining. For EV loading with DNase I and peptide tagging, EVs from NIH3T3 (4x107) cells were isolated and permeabilized with 0,2% saponin for 15 min, RT. EVs were then incubated with 30 units of DNase I and the chimeric peptide (3518, 1µgr/1µgr EVs): CRHSQMTVTSRLRKLRSLWRR at 4°C for 4 hrs. For the EV to EV fusion experiment, EVs were isolated as described above, tagged, loaded, labelled and incubated at 4°C for 4 hrs.

**Rotarod assay.** During the Rotarod test, mice need to keep their balance on a rotating rod by walking forward. One day before testing, mice were trained at a constant rotating mode of 5 rpm for 2 min. During tests, mice were initially placed in their lanes, with the rod rotating at 5 rpm constant speed to allow their positioning. Once all mice were able to move forward, the acceleration test was performed, in which the rod accelerated from 5 rpm to 70 rpm in 60 sec. The time (latency) it took each mouse to fall off the rod rotating under continuous acceleration (from 5 to 70 rpm) was recorded, as well as the reason for trail end (e.g. falling, jumping). The temperature, humidity, ventilation, noise intensity and lighting intensity were maintained constant and at levels appropriate for mice. All mice were kept in a uniform environment before and after testing to avoid anomalous results being obtained.

**Flow cytometry.** Cells and EVs from Cx3CR1-*Ercc1*^{-/-} and wt animals were isolated and stained with fluorochrome conjugated antibodies for 20 min at 4°C in PBS/5% FBS. Live cells were also stained for Annexin V/PI using the FITC Annexin V Apoptosis Detection Kit (556547, BD Pharmingen). Samples were acquired on a FACS Calibur (BD Biosciences) and analyzed using the FlowJo software (Tree Star).

### EXAMPLE 4

Besides microglial cells, EVs derived from Cx3CR1-*Ercc1*^{-/-} microglia also carry dsDNA moieties triggering proinflammatory responses in targeted neurons e.g. Purkinje cells. To remove dsDNA moieties from Cx3CR1-*Ercc1*^{-/-} microglial-derived EVs, we co-incubated the EVs collected from the brain lavages of Cx3CR1-*Ercc1*^{-/-} mice with EVs loaded with DNase I or naive EVs for 4hours at 4°C. Staining with Picogreen (marking the presence of dsDNA) and anti-CD11b followed by flow cytometry analysis revealed that Cx3CR1-*Ercc1*^{-/-}microglial-derived EVs contain substantially less dsDNA load when treated with EVs loaded with DNase I compared to naive EVs. Importantly, we also find lower levels of the circulating Type I IFNs and Picogreen+|CD11b+ EVs in the brain lavages of Cx3CR1-*Ercc1*^{-/-} mice treated with EVs carrying DNase 1 and that Lamin B1 localization was restored.

### Materials and methods:

**Purification and delivery of EVs.** EVs were isolated and loaded with recombinant DNase I as described in example 3. Treatment of cells with EVs containing DNase I nuclease was performed as described in example 3.

**Flow cytometry.**

## Claims

1. An extracellular vesicle comprising a cargo, wherein the cargo comprises
a) a nuclease selected from S1 nuclease, Deoxyribonuclease (DNase) I, Ribonuclease (RNase) H, three prime repair exonuclease (TREX) 1, or
b) mRNA encoding a nuclease selected from S1 nuclease, DNase I, RNase H or TREX 1.

2. The extracellular vesicle according to claim 1, wherein the cargo comprises
a) a nuclease selected from S1 nuclease, DNase I, RNase H nuclease, or
b) mRNA encoding a nuclease selected from S1 nuclease, DNase I, or RNase H.

3. The extracellular vesicle according to claim 1 or 2, wherein the nuclease comprised in the cargo or the nuclease encoded by the mRNA is recombinant.

4. The extracellular vesicle according to any one of the preceding claims, wherein the vesicle has a size from 20 nm to 1000 nm.

5. The extracellular vesicle according to any one of the preceding claims, wherein the vesicle has a size from 30 nm to 200 nm.

6. The extracellular vesicle according to any one of the preceding claims, wherein the vesicle is an exosome.

7. The extracellular vesicle according to any one of the preceding claims, wherein the vesicle comprises on its membrane a cell-penetrating peptide.

8. The extracellular vesicle according to claim 7, wherein the cell-penetrating peptide targets a specific cell type.

9. The extracellular vesicle according to claim 7 or 8, wherein the cell-penetrating peptide consists of the sequence CRHSQMTVTSRLRKLRSLWRR (SEQ ID NO:1).

10. The extracellular vesicle according to any one of the preceding claims, for use in the treatment of an inflammatory disease.

11. The extracellular vesicle for use according to claim 10, wherein the inflammatory disease is chronic or acute.

12. The extracellular vesicle for use according to claim 10 or 11, wherein the inflammatory disease involves inflammation of the brain, pancreas, kidney, liver, lung, skin, or joint.

13. The extracellular vesicle for use according to any one of claims 10 to 12, wherein the inflammatory disease is a neuroinflammatory or neurodegenerative disease, pancreatitis, kidney disease, liver inflammation, inflammatory arthritis, or systemic lupus erythematosus.

14. A pharmaceutical composition comprising as active ingredient an extracellular vesicle as defined in any one of claims 1 to 9 and a pharmaceutically acceptable carrier.

15. The pharmaceutical composition according to claim 14, wherein the composition is suitable for parenteral, intramuscular, intracerebral, intravenous, subcutaneous, transdermal, oral or inhalation administration.
